# EUROPEAN PATENT SPECIFICATION

(11) **EP 2 825 666 B1**
(45) Date of publication and mention of the grant of the patent: **05.12.2018**
(21) Application number: 13710334.7
(22) Date of filing: 08.03.2013
(51) Int. Cl.: C12Q 1/6804

(54) **IMMUNOASSAY FOR DETECTION OF MIRNAS**
IMMUNOASSAY ZUR ERKENNUNG VON MIRNA
DOSAGE IMMUNOLOGIQUE POUR LA DÉTECTION DE MIARN

(30) Priority: 13.03.2012 EP 12159196
(43) Date of publication of application: 21.01.2015
(73) Proprietor: Siemens Healthcare GmbH, 91052 Erlangen (DE); Siemens Healthcare Diagnostics Holding GmbH, 65760 Eschborn (DE); Siemens Healthcare Diagnostics Inc., Tarrytown, NY 10591-5098 (US); Siemens Healthcare Diagnostics Products GmbH, 35041 Marburg (DE)
(72) Inventor: KAPPEL, Andreas, 61479 Glashütten (DE); KELLER, Andreas, 66346 Püttlingen (DE); STÄHLER, Cord Friedrich, 69493 Hirschberg an der Bergstraße (DE); WRIGHT, Ian, Maryland, Maryland 21919 (US); ANDERSON-MAUSER, Linda Marie, Elkhart, Indiana 46517 (US); BEDZYK, William, Odessa, Delaware 19730 (US); SCHWARZ, Herbert, 35102 Lohra (DE); WICKE, Michaela, 34560 Fritzlar (DE)
(74) Representative: Maier, Daniel Oliver
(86) International application number: PCT/EP2013/054745
(87) International publication number: WO 2013/135581

(56) References cited:
- WO-A1-2011/097528
- WO-A2-2006/099537
- STOLLAR B D ET AL: "Immunochemical approaches to gene probe assays", ANALYTICAL BIOCHEMISTRY, ACADEMIC PRESS INC, NEW YORK, vol. 161, no. 2, March 1987 (1987-03), pages 387-394, XP024817717, ISSN: 0003-2697, DOI: 10.1016/0003-2697(87)90467-2 [retrieved on 1987-03-01]
- MATTHEWS J A ET AL: "Analytical strategies for the use of DNA probes", ANALYTICAL BIOCHEMISTRY, ACADEMIC PRESS INC, NEW YORK, vol. 169, no. 1, 15 February 1988 (1988-02-15), pages 1-25, XP024823244, ISSN: 0003-2697, DOI: 10.1016/0003-2697(88)90251-5 [retrieved on 1988-02-15]

## Description

### Field of the invention

The present invention relates to a method and use of a kit for detecting a nucleic acid in a biological sample, wherein the nucleic acid molecule is a microRNA.

### Background of the invention

Very recently, molecular diagnostics has increasingly gained in importance. It has found an entry into the clinical diagnosis of diseases (inter alia detection of infectious pathogens, detection of mutations of the genome, detection of diseased cells and identification of risk factors for predisposition to a disease). However, in the meantime molecular diagnostic methods are also finding their uses in veterinary medicine, environmental analysis and foodstuff testing.

In particular, through the determination of gene expression in tissues, nucleic acid analysis opens up very promising new possibilities in the study and diagnosis of disease.

In order to reduce costs and keep the processing time from sample receipt to determination of the analytical result (also referred to as "time to result") as short as possible, it is a priority target to make methods for detecting nucleic acids as efficient as possible and as far as possible to perform them with automation. This applies particularly to diagnostics. Those methods which can be carried out in reaction vessels differing as little as possible and can be carried out in standardized formats (e.g. 96-well microtiter plate format) are well suited for automation since in these methods efficient pipetting robots can be used. Hence in the state of the art there is the need for simple, efficient and automatable sample analysis.

Today, specific nucleic acid sequences are typically detected or quantified by e.g. heterogenous hybridization assays, PCR methods, or direct sequencing. Those types of assays typically have a quite long time to result, which makes them difficult to apply for critical medical situations e.g. in the course of cardiac events. Moreover, the current tests are not adaptable to immunoassay analyzers, which are the most prominent platforms in the clinical laboratory or in point-of-care settings such as emergency rooms.

Nucleic acids of interest to be detected include genomic DNA, expressed mRNA and other RNAs such as MicroRNAs (abbreviated miRNAs). MiRNAs are a new class of small RNAs with various biological functions (A. Keller et al., Nat Methods. 2011 8(10):841-3). They are short (average of 20-24 nucleotide) ribonucleic acid (RNA) molecules found in eukaryotic cells. Several hundred different species of microRNAs (i.e. several hundred different sequences) have been identified in mammals. They are important for post-transcriptional gene-regulation and bind to complementary sequences on target messenger RNA transcripts (mRNAs), which can lead to translational repression or target degradation and gene silencing. As such they can also be used as biologic markers for research, diagnosis and therapy purposes. In the prior art miRNAs are also detected and quantified by heterogenous hybridization assays or direct sequencing. It is a particular challenge to reliably quantify miRNAs as these are inherently chemically unstable and prone to degradation. This is due to the chemical instability of RNA, the fact that miRNA are present as single strand nucleic acids and due to their short length.

In a different approach, Quavit et al (Anal. Chem., 2011, 83 (15), pp 5949-5956) employ oligo-nucleotide-coated gravimetric sensors to bind miRNAs. The resultant hybrids are bound by anti DNA:RNA antibodies and the mass difference between hybrid and hybrid with bound antibody is gravimetrically detected. As the nucleic acid probes are part of the sensor, this approach is inflexible with regard to target nucleic acid of interests, as an individual sensor needs to be configured for each target nucleic acid of interest.

A further problem is that many applications in molecular-biological research and diagnostics require determining the amount or concentration of particular nucleic acids in a sample. The standard method for quantifying nucleic acids is by PCR reaction, employing an internal standard of known concentration and/or using real-time PCR. Disadvantageously, however, these methods have a limited dynamic range. The typical dynamic range is between 1 to 100 and 1 to 1000, but the range of nucleic acid concentrations to be measured often is distinctly larger, for example 1 to 1 000 000. Further, PCR methods have been perceived as unreliable for quantization due to the possibility of contamination and nonlinear enzyme kinetics.

Stollar B D et al, "Immunochemical approaches to gene probe assays", Analytical Biochemistry, 161, 2 1987, pp. 387-394 discusses the application of antibodies specific for helical nucleic acids to assays for hybridized DNA and/or RNA.

In WO 2011/097528 A1 compositions and methods for detecting target RNA molecules are disclosed.

Matthews J A et al., "Analytical strategies for the use of DNA probes", Analytical Biochemistry, 169, 1, 1988, pp. 1-25 describes various labels and strategies for DNA probe assays which have been employed in both routine and research applications.

Furthermore WO 2006/099537 A2 relates to compositions, methods and kits for labeling proteins and uses in reporter systems for detecting, quantifying and/or characterizing analytes.

### Definitions

Unless defined otherwise, technical and scientific terms used herein have the same meaning as commonly understood by one of ordinary skill in the art to which this invention belongs.

The term "antibody", as used herein, refers to an immunoglobulin protein or a fragment thereof, said fragment being capable of specifically binding an antigen. An antibody, including an antibody fragment, suitable for the invention may be monoclonal, polyclonal, of any host organism source, recombinantly expressed or otherwise artificially produced and of any immunoglobuline type. In the context of the invention, the antibody is able to bind an antigen comprising a hybrid of a nucleic acid molecule of interest and a nucleic acid probe.

The term "hybridization ", as used herein, refers to the process of combining complementary, single-stranded nucleic acids or nucleotide analogues into a single double stranded molecule, the so-called "hybrid". Nucleotides or nucleotide analogues will bind to their complement under normal conditions, so two complementary strands will bind to each other readily. Single stranded probes can be used in order to find complementary target sequences. If such sequences exist in the sample, the probes will hybridize to said sequences which can then be detected.

The term "immunoassay", as used herein, refers to the detection or quantification of an analyte - such as a nucleic acid of interest - comprising an immune reaction between an antibody and an antigen. In the context of the invention the analyte to be detected or quantified comprises a nucleic acid of interest.

A "label moiety" within the meaning of the invention, shall have the ordinary meaning of this term which is well known to the person skilled in the art of molecular biology. The label moiety may comprise a binding partner of a specific binding pair, wherein the respective binding reaction is used to couple the label moiety to a detectable label. Further, the label moiety itself may comprise any detectable label. Detection may be achieved in any suitable fashion, e.g. by optical detection, chemical detection, electrochemical detection, radioactive detection, magnetic detection or any other suitable mode of detection. Examples for label moieties include enzymatic labels, chromogenic labels, fluorescent labels, chemiluminescent labels, radioactive labels, and magnetic labels

The term "marker" or "biomarker" refers to a biological molecule, e.g., a nucleic acid, peptide, protein, hormone, etc., whose presence or concentration can be detected and correlated with a known condition, such as a disease state.

The term "nucleic acid" is intended to indicate any nucleic acid molecule and/or analogous molecules comprised of a sequence of nucleotides including DNA, cDNA and/or genomic DNA, RNA, preferably miRNA, peptide nucleic acid (PNA), locked nucleic acid (LNA) and/or morpholino.

"Nucleic acid of interest", within the meaning of the invention refers to a target nucleic acid which is to be detected and/or quantified. In particular, this term refers to a nucleic acid having a specific predetermined sequence.

"Nucleic acid probes", within the meaning of the invention, shall have the ordinary meaning of this term which is well known to the person skilled in the art of molecular biology. In a preferred embodiment of the invention they shall be understood as being polynucleotide molecules having a sequence identical, complementary, or homologous to the complement of regions of a target nucleic acid of interest which is to be detected or quantified. In yet another embodiment, nucleotide analogues are also comprised for usage as nucleic acid probes.

The terms "sample", "biological sample", or "clinical sample", as used herein, refer to any sample containing a nucleic acid of interest. A sample may be obtained from a patient. The sample may be of any biological tissue or fluid. Such samples include, but are not limited to, sputum, blood, serum, plasma, blood cells (e.g., white cells), tissue, biopsy samples, smear samples, lavage samples, swab samples, cell-containing body fluids, free floating nucleic acids, urine, peritoneal fluid, and pleural fluid, liquor cerebrospinalis, urine, feces, tear fluid, or cells therefrom. Biological samples may also include sections of tissues such as frozen or fixed sections taken for histological purposes or microdissected cells or extracellular parts thereof.

The term "solid phase", as used herein, refers to an object which consists of a porous and/or nonporous, material which is insoluble in the solvent system used (e.g. water insoluble). It can have a wide variety of forms such as vessels, tubes, plates, spheres, microparticles, rods, strips, filter paper, chromatography paper, etc.. The term "solid phase bound" refers to an object, such as an antibody, being attached to the solid phase in such a way that it will not become detached and dissolved into solution when the methods of the invention described herein are performed. Binding may be achieved for example by physissorbtion, chemisorption, covalent linking, as is known in the art.

Object of the invention

The technical problem underlying the present invention is to provide a method for the detection of nucleic acid molecules which is easily adaptable to nucleic acid molecules with different sequences and which is easily adaptable to automated laboratory platforms or point-of care type settings.

It is a further object of the invention to provide a method for the detection of nucleic acid molecules which allows to obtain quantifiable results with an improved dynamic range. It is another object of the present invention to provide a method suitable for the detection and/or quantification of miRNAs.

It is another object of the present invention to provide a use of a kit for the detection of nucleic acid molecules meeting the above criteria.

These objects are met with the methods and means according to the independent claims of the present invention. The dependent claims are related to preferred embodiments.

### Summary of the invention

Before the invention is described in detail, it is to be understood that this invention is not limited to the particular component parts of the process steps of the methods described as such methods may vary. It is also to be understood that the terminology used herein is for purposes of describing particular embodiments only, and is not intended to be limiting. It is moreover to be understood that, in case parameter ranges are given which are delimited by numeric values, the ranges are deemed to include these limitation values.

The invention relates to a method of detecting nucleic acid molecules by immunoassay detection, wherein the nucleic acid molecules are microRNAs. Detection of a specific nucleic acid molecule of interest is achieved by using a nucleic acid probe in solution which hybridizes in solution to the nucleic acid molecule of interest. Immunoassay detection is achieved by using an antibody specific for the hybrid formed by hybridization of the nucleic acid probe to the nucleic acid molecule of interest.

In the most general terms, the invention relates to a method of detecting nucleic acid molecules by immunoassay detection, wherein the nucleic acid molecules are microRNAs. Detection of a specific nucleic acid molecule of interest is achieved by using a dissolved nucleic acid probe which hybridizes to the nucleic acid molecule of interest. Immunoassay detection is achieved by an antibody specific for the hybrid formed by hybridization of the nucleic acid probe to the nucleic acid molecule of interest. The antibody is provided in a form which is bound to a solid phase. Alternatively described is that the antibody can be provided in solution and the nucleic acid of interest can be detected in a homogeneous immunoassay. The hybrid can be provided in solution. This solution can be brought into contact with the antibody. The antibody will bind the hybrid and the complex of antibody and bound hybrid can be detected. This allows easy adaptation to nucleic acid molecules with different sequences and easy adaptation to automated laboratory platforms or point-of care type settings and devices. Such an antibody can e.g. be obtained by immunizing a host organism with the respective hybrid (e.g. a RNA:DNA hybrid) and collecting antibodies or antibody-producing cells, as is known in the art.

The method and use of a kit of the invention allow a fast and homogenous detection and quantification of specific nucleic acid sequences such as miRNAs.

As immunoassays typically have short times to result, and are adaptable to immunoassay analyzers, this novel assay format overcomes the problems of the current test formats mentioned above.

In particular, the invention relates to the method of claim 1 and the use of a kit of claim 7.

To meet the need for a flexible assay system for the detection and/or quantification of miRNAs in e.g. a clinical laboratory setting, the present invention provides a method for the immunoassay detection of miRNAs. This provides an alternative to existing methods. Immunoassays typically have short times to result, and are adaptable to automated immunoassay analyzers.

In an aspect the invention relates to a method for detecting a nucleic acid molecule of interest in a sample, wherein the nucleic acid molecule of interest is a microRNA, comprising the following steps:
- providing a nucleic acid probe in solution, wherein the nucleic acid probe is labelled with a label moiety;
- hybridizing the nucleic acid molecule of interest to the nucleic acid probe to obtain hybrid of said nucleic acid molecule of interest and said nucleic acid probe;
- binding said hybrid to an antibody capable of specifically binding nucleic acid hybrids, wherein the antibody is bound to a solid phase; and
- detecting the antibody-bound hybrid, wherein a plurality of different nucleic acids of interest is detected by using a respective plurality of nucleic acid probes,
wherein different nucleic acid probes are used in different wells of a microtiter plate to detect different nucleic acids of interest, wherein the microtiter plate is pre-coated with the antibody, wherein a temperature gradient across the field of the microtiter plate is applied.

Further described is a method for detecting a nucleic acid molecule of interest in a sample, comprising the following steps:
- providing a nucleic acid probe in solution;
- hybridizing the nucleic acid molecule of interest to the nucleic acid probe to obtain hybrids of said nucleic acid molecule of interest and said nucleic acid probe;
- binding said hybrid to an antibody capable of specifically binding nucleic acid hybrids, wherein the antibody is bound to a solid phase; and
- detecting the antibody-bound hybrid

Binding of the antibody to the hybrid is achieved by bringing the solid-phase bound antibody into contact with the solution containing the hybrid. Because one of the binding partners forming the detectable complex of antibody and hybrid is solid-phase bound, this aspect of the invention can be referred top as a "hetereogeneous" format. A particular advantage of this format lies in the ability to provide a solid phase with a bound antibody, wherein the antibody serves as a universal capture moiety for the hybrids. This allows for convenient assay formats, where the antibody is pre-coated onto microtiter plates.

Further described is a method for detecting a nucleic acid molecule of interest in a sample, comprising the following steps:
- providing a nucleic acid probe in solution;
- hybridizing the nucleic acid molecule of interest to the nucleic acid probe to obtain hybrids of said nucleic acid molecule of interest and said nucleic acid probe;
- binding said hybrid to an antibody capable of specifically binding nucleic acid hybrids, wherein the antibody is in solution; and
- detecting the antibody-bound hybrid in solution.

Binding of the antibody to the hybrid therein can be achieved by bringing the antibody in solution into contact with the solution containing the hybrid. Because of both binding partners forming the detectable complex of antibody and hybrid being in solution, this can be referred to as a "homogeneous" format.

In both the homogeneous and the heterogeneous format, the nucleic acid probe is provided in solution and hybridization takes place in solution. This makes both formats easily adaptable to the detection of different nucleic acids of interest (by selecting the nucleic acid probe accordingly), and it also makes both formats easily adaptable to automated analysis systems such as are commonly used in clinical laboratories.

According to certain aspects the antibody is bound to a competitive antigen prior to the binding of said first hybrid and said hybrid is detected by displacement of the competitive antigen.

The competitive antigen can be a competitive hybrid of nucleic acids. Further, the competitive hybrid can carry a label moiety. Well known detection technologies for competitive immunoassays, such as FRET (fluorescence resonance energy transfer) or EMIT (Enzyme multiplied immunoassay technique), can be employed.

Advantageously, the method of the invention allow the detection of many different species (i.e. having different sequences) of nucleic acid of interest with the same antibody serving as a universal capture moiety. Specific detection of an individual species is achieved by using a nucleic acid probe which will hybridize to the individual species of the nucleic acid of interest. Further, advantageously, these methods offer a high dynamic range. Immunoassays routinely offer a dynamic range from 1:1000 to 1:100000 which can easily be increased further by performing a simple dilution series.

Described is that the nucleic acid molecule of interest is an RNA. In this case it is also contemplated that the RNA can be converted to complementary DNA (cDNA) prior to performing the method.

According to certain embodiments the nucleic acid probe is a DNA.

According to certain embodiments the antibody is specific for DNA/RNA hybrids

The nucleic acid probe is labelled with a label moiety. According to a preferred embodiment the label moiety is provided by biotinylation of the nucleic acid probe. This allows detection by using a detectable label coupled to streptavidin.

According to certain embodiments the labeling moiety comprises an enzyme. A plurality of enzyme-substrate detection chemistries are commercially available. They are well established both for e.g. microtiter-plate formats micro-particle formats and test strip formats and are compatible with commercially available analyzer systems and testing devices and their detectors. According to a preferred embodiment the label moiety is provided by biotinylation of the nucleic acid probe and use of a strepatavidin-enzyme conjugate as detectable label. The use of an enzymatic label offers a further path to increasing dynamic range. As a detecable product is developed in an accumulating enzymatically catalyzed reaction between enzyme and substrate(s), the sample can be read when the reaction has developed to a degree where signal is well over background and within the linear range of the detection system. Many enzyme-substrate chemistries further offer the possibility to use stop solutions which usually comprises the addition of a substance that will stop enzymatic activity. In this way, the enzymatically catalyzed reaction can be stopped once it has progressed to an appropriate degree and the sample can be read either immediately or at a later point in time without the danger of "overdeveloping".
The nucleic acid molecule of interest is a miRNA. As several hundred miRNA species are known in humans, the method and use of a kit of the invention are particularly well suited for the detection and quantification of miRNAs. Simply by providing the appropriate nucleic acid probe it is possible to adapt the method and use of a kit of the invention for the detection of any number of miRNAs.

According to certain embodiments the detection is a quantitative detection. If desired, the concentration can be measured as an absolute concentration, for example, by using an internal standard (a known nucleic acid with a known initial concentration, which is added to the sample or is amplified and measured in parallel). In many cases it may be sufficient to determine the relative concentration of the nucleic acid of interest in the test sample against a reference sample, e.g. by comparing a patient sample against a sample from a healthy control.
A plurality of different nucleic acids of interest are detected by using a respective plurality of nucleic acid probes.

The invention further relates to a use of a kit for carrying out a method of any one of the aforementioned claims, wherein the kit comprises at least a plurality of labeled nucleic acid probes in solution or in a dissolvable form and an antibody capable of binding a hybrid of said nucleic acid molecule of interest to said plurality of labeled nucleic acid probes, wherein the antibody is bound to a solid phase, further comprising a support for supporting the solid phase to which the antibody is bound, wherein the support comprises a microtiter plate. Also described is a kit for detecting a nucleic acid molecule of interest in a sample, said kit being useful for carrying out the methods, comprising at least a nucleic acid probe in solution or in a dissolvable form and an antibody capable of binding a hybrid of said nucleic acid molecule of interest to said nucleic acid probe.

In the kit, the nucleic acid probe can be provided in a solution, either as a working solution or as a dilutable stock solution. Alternatively it can be provided in a dissolvable form, for example in lyophilized form.

The kit may further comprise any or all of the following: controls, standards, calibrators developing solutions for developing detectable labelling reactions, stop solutions for stopping development of a labelling reactions, blocking solutions for blocking unspecific binding or hybridization, wash solutions for reduction of unspecific background signal etc., as is known in the art.

The kit can further comprise the antibody bound to a solid phase. A particular advantage of this type of kit lies in the ability to provide a solid phase with a bound antibody, wherein the antibody serves as a universal capture moiety for the hybrids. This allows for convenient assay formats, where the antibody is pre-coated onto commonly used sold phase formats such as microtiter plates, test strips, articles and the like.

The kit can further comprise a support for supporting the solid phase to which the antibody is bound.

The solid phase can have a very wide variety of forms, for example those of vessels, tubes, microtitration plates, spheres, microparticles, rods, strips, filter paper, chromatography paper, etc. As a rule, the surface of the solid phase is hydrophilic or can be made hydrophilic. The solid phase can consist of a very wide variety of materials, for example of inorganic and/or organic materials, of synthetic materials, of naturally occurring materials and/or of modified naturally occurring materials. Examples of solid phase materials are polymers, such as cellulose, nitrocellulose, cellulose acetate, polyvinyl chloride, polyacrylamide, crosslinked dextran molecules, agarose, polystyrene, polyethylene, polypropylene, polymethacrylate or nylon; ceramic, glass or metals, in particular precious metals such as gold and silver; magnetite; mixtures or combinations thereof; etc. Cells, liposomes and phospholipid vesicles are also covered by the term solid phase.

The solid phase can also possess a coating consisting of one or more layers, for example of proteins, carbohydrates, lipophilic substances, biopolymers or organic polymers, or mixtures thereof, in order, for example, to suppress or prevent the nonspecific binding of sample constituents to the solid phase or in order, for example, to achieve improvements with regard to the suspension stability of particular solid phases, with regard to storage stability, with regard to dimensional stability or with regard to resistance to UV light, microbes or other agents having a destructive effect.

The support can comprise a microtiter plate.

The support can also comprise a test strip. The test strip can be of a simple dip format, wherein the strip is dipped into a sample fluid. It can also be embedded in a cassette, wherein a drop of sample fluid is transferred into an appropriate opening of the cassette.

It is to be understood that the support itself or a portion thereof may serve as solid-phase to which the antibody is bound. In the case of the support being a microtiter plate with at least one or a plurality of wells, the antibody can be coated on the interior surface (or on a portion thereof) of the well(s). In the case of the support being a test strip, the antibody can be coated on a portion of the test strip, e.g. on one or several distinct bands on the strip thus yielding a corresponding pattern of labelled bands when the test is performed.

The support may have distinct portions dedicated to controls, as is known in the art.

According to a preferred embodiment of the invention the method and use of kit can be in an ELISA format (Enzyme-linked immunosorbent assay). This format essentially combines the use of a solid-phase bound antibody with the use of an enzymatic label, i.e. a label moiety comprising an enzyme. Preferably, suitable microtiter plates are coated with the antibody which will bind the hybrid. Described is that a test strip or similar simple solid-phase device is pre-coated with the antibody which will bind the hybrid. This form is well suited for use in a point-of care setting, e.g. in the emergency room.
The nucleic acid probe is labelled. In this way the hybrid will be easily detectable allowing for a simple, straightforward assay format. Alternatively, the complex of antibody and hybrid may be detected by a further suitable secondary antibody in a type of Sandwich-immunoassay format.

The kit comprises the antibody in a solid phase-bound form on pre-coated microtiter plates. In this way multiplex assay is easily possible, where a combination of the solid phase-bound antibody with a plurality of nucleic acid probes can be used to detect a corresponding plurality of nucleic acids of interest. A microtiter plate, such as a 96-well plate, is pre-coated with the antibody and different nucleic acid probes are used in different wells to detect different nucleic acids of interest. Further described is an alternative embodiment, wherein different nucleic acid probes with different label moieties may be used in the same assay to detect different nucleic acids of interest. By using an antibody bound to a solid phase as a universal capture moiety for hybrids comprising different combinations of nucleic acids of interest and respective nucleic acid probes a plurality of assays can be performed in parallel, e.g. using the same solid phase, such as an antibody-coated microtiter plate. Hybridization can be e.g. performed in a PCR machine or similar heating device which allows to select a predetermined temperature for hybridization. If such a heating device allows the application of a temperature gradient across the field of the microtiter plate, an optimal hybridization temperature can be quickly determined. Moreover, by using an antibody bound to a solid phase as a universal capture moiety it then becomes possible to perform hybridizations with a plurality of nucleic acid probes at a corresponding plurality of differing optimal hybridization temperatures. This can be done e.g. in separate wells of the same microtiter plate.

The method of the invention, wherein the antibody as a universal capture moiety for hybrids is provided in a solid phase-bound form and the hybrid (comprising the nucleic acid probe) as capture target is added in solution thus offers many advantages in terms of both adapting the method to multiplex assays and adapting the method to existing analyzer systems, devices, and workflows.

The antibody is bound to a solid phase and the nucleic acid probe is labelled with a labelling moiety. This provides for a straightforward assay protocol and short time to result.

The method and use of a kit of the invention provide a simple, specific, and sensitive way to detect a nucleic acid of interest which is easily adaptable to existing automated analyzer systems such as are used in central laboratory of hospitals or doctor's offices.

The method and use of a kit of the invention has proven to be particularly useful for the detection of miRNAs. They allow a fast and accurate detection which can be carried out on automated analyzer systems.

### Brief description of the examples

Additional details, features, characteristics and advantages of the object of the invention are further disclosed in the following description of the respective examples, which, in an exemplary fashion, show preferred embodiments of the present invention. However, these examples should by no means be understood as to limit the scope of the invention.

Briefly, in a first step, a defined quantity of biotin labelled sequence-specific DNA oligonucleotide is hybridized to the miRNA from e.g. a patient sample under conditions that only a single miRNA species can hybridize. The amount of RNA:DNA hybrids generated during this step is proportional to the amount of the specific miRNA species present in that sample. In a second step, the hybridisation mixture is transferred to a solid phase coated with a mAb to RNA:DNA hybrids; any hybrids present will bind to the solid phase. Binding of RNA:DNA hybrids is detected by a suitable label system e.g. Streptavidin-peroxidase (Streptavidin-POD). The amount of POD bound is proportional to the amount of RNA:DNA hybrids present in the original sample.

Detection and quantification of miRNA is inherently difficult. Surprisingly and unexpectedly, it has been found that this method allows for a highly specific, sensitive and reproducible detection of miRNAs. Without wishing to be bound to this theory, inventors suspect that this may be due to stabilization of miRNAs in a double-stranded hybrid in solution, in particular in RNA:DNA heteroduplexes. It can be shown that the hybrids in solution can be frozen, stored for long periods of time and subsequently still be detected with high sensitivity in the method of the invention.

In an experiment the miRNAs miR-7, miR-380 or miR-1254 were hybridized to complementary DNA oligonucleotides, or to a non-specific DNA oligonucleotide as control. After hybridization, the DNA:RNA hybrid solutions were transferred to the wells of an ELISA plate that was pre-coated with an antibody to DNA:RNA hybrids. After washing steps and detection of antibody-bound DNA:RNA hybrids by Streptavidin-peroxidase, the peroxidase activity of each well was tested.

The miRNAs miR-7, miR-380 and miR-1254 were used as the targeted nucleic acids of interest. Each miRNA was hybridized with three different nucleic acid probes specific to the respective miRNA of interest. Hybridization was performed at varying temperatures as indicated in table 2 below. As control, the miRNA was also incubated with a non-specific nucleic acid probe under identical hybridization conditions. The nucleic acid probes were labelled with biotin.

The miRNAs were diluted to 100pmol/µl in a suitable storage buffer (such as Tris HCl/EDTA (TE) buffer) and synthetic DNA oligonucleotides were diluted to 100pmol/µl in 1x TrisHCl saline buffer (TBS). 30µl RNA solution and 30µl DNA solution were incubated for 2 min at 95°C to dissolve secondary structures and then hybridized at 40-65°C. After that, hybrids could be used immediately or frozen (e.g. at -20°C) and stored for later use.

The sequences for the nucleic acid probes and miRNAs are given in the table 1 below:

**Table 1: Sequences of nucleic acid probes and miRNAs**

| SEQ ID NO | Nucleic Acid | Sequence (5'-3') |
|---|---|---|
| 1 | miR-7 | UGGAAGACUAGUGAUUUUGUUGU |
| 2 | miR7 Oligo 1 | AACAAAATCACTAGTCTTCCA |
| 3 | miR7 Oligo 2 | AAAATCACTAGTCTTCCA |
| 4 | miR7 Oligo 3 | ATCACTAGTCTTCCA |
| | | |
| 5 | miR-380 | UAUGUAAUAUGGUCCACAUCUU |
| 6 | miR380 Oligo 1 | AGATGTGGACCATATTACATA |
| 7 | miR380 Oligo 2 | TGTGGACCATATTACATA |
| 8 | miR380 Oligo 3 | GGACCATATTACATA |
| | | |
| 9 | miR-1254 | AGCCUGGAAGCUGGAGCCUGCAGU |
| 10 | miR1254 Oligo 1 | GCAGGCTCCAGCTTCCAGGCT |
| 11 | miR1254 Oligo 2 | GGCTCCAGCTTCCAGGCT |
| 12 | miR1254 Oligo 3 | TCCAGCTTCCAGGCT |

ELISA plates were pre-coated with an anti-DNA:RNA-Hybrid antibody under standard conditions(5µg/ml in phosphate-buffered saline (PBS)). After coating, the ELISA plates were washed with a commercially available wash solution or a TRIS/citric acid buffer.

The hybrids were diluted 1: 100, 1: 1000, 1:10000 and 1:100000 in
- PBS, -
- PBS with 1 µg/ml BSA and 0,1 µg/ml Tween 20,
- PBS with 0,5 µg/ml BSA and 0,05 µg/ml Tween 20, or
- PBS with 0,25 µg/ml BSA and 0,025 µg/ml Tween 20 Respectively and incubated on coated ELISA plates for 1 hr at 37°C. After incubation plates were washed and incubated with a commercially available Streptavidine POD conjugate. Plates were washed and a chromogenic substrate (tetramethyl benzidine) solution was added to the plates. Color development was stopped by addition of a sulphuric acid stop solution and optical density (OD) was read at 450 nm. Results for 1:100000 dilutions of hybrids 1 to 12 are given in table 2, below.

**Table 2: OD Values**

| Hybrid Composition | Hybrid No. | Diluent | | | |
|---|---|---|---|---|---|
| | | 1xPBS | 1xPBS, 1g/l BSA, 0,1g/l Tween 20 | 1xPBS 0,5g/l BSA, 0,05g/l Tween 20 | 1xPBS 0,25g/l BSA, 0,025g/l Tween 20 |
| 1. RNA miR-7 with DNA miR7 Oligo 1, hybridisation temperature 52,0°C | Hybrid 1 | 3.094 | 3.289 | 3.275 | 3.325 |
| 2. RNA miR-7 with DNA miR7Oligo 2, hybridisation temperature 46,9°C | Hybrid 2 | 3.079 | 3.306 | 3.259 | 3.259 |
| 3. RNA miR-7 with DNA miR7 Oligo 3, hybridisation temperature 42,4 °C | Hybrid 3 | 2.517 | 3.164 | 3.004 | 2.980 |
| 4. RNA miR-7 with DNA miR380 Oligo 1, hybridisation temperature 52,0°C | Hybrid 4 (non-specific) | 0.021 | 0.028 | 0.022 | 0.022 |
| 5. RNA miR-380 with DNA miR380 Oligo 1, hybridisation temperature 52,0°C | Hybrid 5 | 2.952 | 3.104 | 3.104 | 3.074 |
| 6. RNA miR-380 with DNA miR380 Oligo 2, hybridisation temperature 46,9°C | Hybrid 6 | 2.912 | 3.254 | 3.173 | 3.138 |
| 7. RNA miR-380 with DNA miR380 Oligo 3, hybridisation temperature 39,6°C | Hybrid 7 | 1.046 | 2.109 | 2.128 | 1.817 |
| 8. RNA miR-380 with DNA miR7 Oligo 1, hybridisation temperature 52,0°C | Hybrid 8 (non-specific) | 0.023 | 0.029 | 0.031 | 0.030 |
| 9. RNA miR-1254 with DNA miR1254 Oligo 1, hybridisation temperature 65,7°C | Hybrid 9 | 3.139 | 3.244 | 3.270 | 3.215 |
| 10.RNA miR-1254 with DNA miR1254 Oligo 2, hybridisation temperature 60,5°C | Hybrid 10 | 3.003 | 3.230 | 3.259 | 3.217 |
| 11.RNA miR-1254 with DNA miR1254 Oligo 3, hybridisation temperature 50,6°C | Hybrid 11 | 2.554 | 3.164 | 3.106 | 3.067 |
| 12.RNA miR-1254 with DNA miR7 Oligo 1, hybridisation temperature 52,0°C | Hybrid 12 (non-specific) | 0.027 | 0.033 | 0.030 | 0.030 |
| 1xPBS | (background) | 0.028 | 0.032 | 0.031 | 0.030 |

As can easily be seen from the results shown in table 2, the method of the invention allows for an accurate, sensitive, specific and reproducible detection of miRNAs.

All full complementary DNA:RNA hybrids tested resulted in ODs >>1, whereas unspecific DNA:RNA hybrids or buffer alone resulted in ODs <<0.1. The OD values for non-specific controls (i.e. hybrids 4, 8 and 12) were in the same order of magnitude as zero values obtained with only PBS instead of a hybrid sample. It has thus been shown for the first time that miRNAs can be detected with high sensitivity and specificity by an immunoassay method. The assay described in the above example has been repeatedly performed with varying combinations of buffers and washing solutions, all with comparable results, thus demonstrating a robust, reproducible method of detecting miRNAs. Dilution series show a good detection over several orders of magnitude with high linearity and reproducibility.

The ELISA assay format as used e.g. in the above example is known to be well suited for a quantitative detection of analytes as well.

## Claims

1. A method for detecting a nucleic acid molecule of interest in a sample, wherein the nucleic acid molecule of interest is a microRNA, comprising the following steps:
- providing a nucleic acid probe in solution, wherein the nucleic acid probe is labelled with a label moiety;
- hybridizing the nucleic acid molecule of interest to the nucleic acid probe to obtain hybrid of said nucleic acid molecule of interest and said nucleic acid probe;
- binding said hybrid to an antibody capable of specifically binding nucleic acid hybrids, wherein the antibody is bound to a solid phase; and
- detecting the antibody-bound hybrid, wherein a plurality of different nucleic acids of interest is detected by using a respective plurality of nucleic acid probes,
wherein different nucleic acid probes are used in different wells of a microtiter plate to detect different nucleic acids of interest, wherein the microtiter plate is pre-coated with the antibody, wherein a temperature gradient across the field of the microtiter plate is applied.

2. The method according to claim 1, wherein the antibody is bound to a competitive antigen prior to the binding of said first hybrid and said hybrid is detected by displacement of the competitive antigen.

3. The method according to claim 2, wherein the competitive antigen is a competitive hybrid of nucleic acids, said competitive hybrid carrying a label moiety.

4. The method according to any of the aforementioned claims, wherein the nucleic acid probe is a DNA.

5. The method according to claim 4, wherein the antibody is specific for DNA:RNA hybrids.

6. The method according to any one of the aforementioned claims, wherein the detection is a quantitative detection.

7. Use of a kit for carrying out a method of any one of the aforementioned claims, wherein the kit comprises at least a plurality of labeled nucleic acid probes in solution or in a dissolvable form and an antibody capable of binding a hybrid of said nucleic acid molecule of interest to said plurality of labeled nucleic acid probes, wherein the antibody is bound to a solid phase, further comprising a support for supporting the solid phase to which the antibody is bound, wherein the support comprises a microtiter plate.

## Patentansprüche

1. Verfahren zum Nachweisen eines interessierenden Nukleinsäuremoleküls in einer Probe, wobei es sich bei dem interessierenden Nukleinsäuremolekül um eine microRNA handelt, umfassend die folgenden Schritte:
- Bereitstellen einer Nukleinsäuresonde in Lösung, wobei die Nukleinsäuresonde mit einer Markierungsgruppierung markiert ist;
- Hybridisieren des interessierenden Nukleinsäuremoleküls an die Nukleinsäuresonde, so dass ein Hybrid des interessierenden Nukleinsäuremoleküls und der Nukleinsäuresonde erhalten wird;
- Binden des Hybrids an einen Antikörper mit der Fähigkeit zur spezifischen Bindung von Nukleinsäurehybriden, wobei der Antikörper an eine Festphase gebunden ist; und
- Nachweisen des antikörpergebundenen Hybrids, wobei eine Vielzahl von verschiedenen Nukleinsäuren von Interesse durch Verwenden einer entsprechenden Vielzahl von Nukleinsäuren nachgewiesen wird, wobei verschiedene Nukleinsäuresonden in verschiedenen Mulden einer Mikrotiterplatte zum Nachweisen von verschiedenen interessierenden Nukleinsäuren verwendet werden, wobei die Mikrotiterplatte mit dem Antikörper vorbeschichtet ist, wobei ein Temperaturgradient über den Bereich der Mikrotiterplatte hinweg angewendet wird.

2. Verfahren nach Anspruch 1, wobei der Antikörper vor der Bindung des ersten Hybrids an ein kompetitives Antigen gebunden ist und das Hybrid anhand der Verdrängung des kompetitiven Antigens nachgewiesen wird.

3. Verfahren nach Anspruch 2, wobei es sich bei dem kompetitiven Antigen um ein kompetitives Hybrid von Nukleinsäuren handelt, wobei das kompetitive Hybrid eine Markierungsgruppierung trägt.

4. Verfahren nach einem der obengenannten Ansprüche, wobei es sich bei der Nukleinsäuresonde um eine DNA handelt.

5. Verfahren nach Anspruch 4, wobei der Antikörper für DNA:RNA-Hybride spezifisch ist.

6. Verfahren nach einem der obengenannten Ansprüche, wobei es sich bei dem Nachweis um einen quantitativen Nachweis handelt.

7. Verwendung eines Kits zur Durchführung eines Verfahrens nach einem der obengenannten Ansprüche, wobei das Kit mindestens eine Vielzahl von markierten Nukleinsäuresonden in Lösung oder in einer auflösbaren Form und einen Antikörper mit der Fähigkeit zum Binden eines Hybrids aus dem interessierenden Nukleinsäuremolekül mit der Vielzahl von markierten Nukleinsäuresonden umfasst, wobei der Antikörper an eine Festphase gebunden ist, ferner umfassend einen Träger zum Tragen der Festphase, an welche der Antikörper gebunden ist, wobei der Träger eine Mikrotiterplatte umfasst.

## Revendications

1. Procédé de détection d'une molécule d'acide nucléique à laquelle on s'intéresse dans un échantillon, dans lequel la molécule d'acide nucléique à laquelle on s'intéresse est un micro ARN, comprenant les stades suivants :
- on se procure une sonde d'acide nucléique en solution, la sonde d'acide nucléique étant marquée par un radical de marquage;
- on hybride la molécule d'acide nucléique à laquelle on s'intéresse à la sonde d'acide nucléique pour obtenir un hybride de la molécule d'acide nucléique à laquelle on s'intéresse et de la sonde d'acide nucléique;
- on fixe l'hybride à un anticorps apte à fixer spécifiquement des hybrides d'acide nucléique, l'anticorps étant fixé à une phase solide; et
- on détecte l'hybride fixé à l'anticorps, une pluralité d'acides nucléiques différents auxquels on s'intéresse étant détectée en utilisant une pluralité respective de sondes d'acide nucléique,
dans lequel on utilise des sondes d'acide nucléique différentes dans des puits différents d'une plaque de microtitrage, la plaque de microtitrage étant pré-revêtue de l'anticorps, un gradient de température étant appliqué dans le champ de la plaque de microtitrage.

2. Procédé suivant la revendication 1, dans lequel on fixe l'anticorps à un antigène compétitif avant la fixation du premier hybride et on détecte l'hybride par un déplacement de l'antigène compétitif.

3. Procédé suivant la revendication 2, dans lequel l'antigène compétitif est un hybride compétitif d'acide nucléique, l'hybride compétitif portant un radical de marquage.

4. Procédé suivant l'une quelconque des revendications précédentes, dans lequel la sonde d'acide nucléique est un ADN.

5. Procédé suivant la revendication 4,
dans lequel l'anticorps est spécifique pour des hybrides d'ADN:ARN.

6. Procédé suivant l'une quelconque des revendications précédentes, dans lequel la détection est une détection quantitative.

7. Utilisation d'une trousse pour effectuer un procédé suivant l'une quelconque des revendications précédentes, la trousse comprenant
au moins une pluralité de sondes d'acide nucléique marquées en solution ou sous une forme dissolvable et
un anticorps capable de fixer un hybride de la molécule d'acide nucléique à laquelle on s'intéresse à la pluralité de sondes d'acide nucléique marquées, l'anticorps étant fixé à une phase solide, comprenant en outre un support pour supporter la phase solide auquel l'anticorps est fixé, le support comprenant une plaque de microtitrage.


### SEQUENCE LISTING

<110> Siemens AG
<120> Immunoassay for detection of specific nucleic acid sequences such as
   miRNAs
<130> 201204166
<160> 12
<170> BiSSAP 1.0
<210> 1
   <211> 23
   <212> RNA
   <213> Homo sapiens
<220>
   <221> source
   <222> 1..23
   <223> /mol_type="RNA" /organism="Homo sapiens"
<400> 1
   uggaagacua gugauuuugu ugu 23
<210> 2
   <211> 21
   <212> DNA
   <213> Artificial
<220>
   <221> source
   <222> 1..21
   <223> /mol_type="DNA" /note="oligonucleotide" /organism="Artificial"
<400> 2
   aacaaaatca ctagtcttcc a 21
<210> 3
   <211> 18
   <212> DNA
   <213> Artificial
<220>
   <221> source
   <222> 1..18
   <223> /mol_type="DNA" /note="oligonucleotide" /organism="Artificial"
<400> 3
   aaaatcacta gtcttcca 18
<210> 4
   <211> 15
   <212> DNA
   <213> Artificial
<220>
   <221> source
   <222> 1..15
   <223> /mol_type="DNA" /note="oligonucleotide" /organism="Artificial"
<400> 4
   atcactagtc ttcca 15
<210> 5
   <211> 22
   <212> RNA
   <213> Homo sapiens
<220>
   <221> source
   <222> 1..22
   <223> /mol_type="RNA" /organism="Homo sapiens"
<400> 5
   uauguaauau gguccacauc uu 22
<210> 6
   <211> 21
   <212> DNA
   <213> Artificial
<220>
   <221> source
   <222> 1..21
   <223> /mol_type="DNA" /note="oligonucleotide" /organism="Artificial"
<400> 6
   agatgtggac catattacat a 21
<210> 7
   <211> 18
   <212> DNA
   <213> Artificial
<220>
   <221> source
   <222> 1..18
   <223> /mol_type="DNA" /note="oligonucleotide" /organism="Artificial"
<400> 7
   tgtggaccat attacata 18
<210> 8
   <211> 15
   <212> DNA
   <213> Artificial
<220>
   <221> source
   <222> 1..15
   <223> /mol_type="DNA" /note="oligonucleotide" /organism="Artificial"
<400> 8
   ggaccatatt acata 15
<210> 9
   <211> 24
   <212> RNA
   <213> Homo sapiens
<220>
   <221> source
   <222> 1..24
   <223> /mol_type="RNA" /note="miR-1254" /organism="Homo sapiens"
<400> 9
   agccuggaag cuggagccug cagu 24
<210> 10
   <211> 21
   <212> DNA
   <213> Artificial
<220>
   <221> source
   <222> 1..21
   <223> /mol_type="DNA" /note="oligonucleotide" /organism="Artificial"
<400> 10
   gcaggctcca gcttccaggc t 21
<210> 11
   <211> 18
   <212> DNA
   <213> Artificial
<220>
   <221> source
   <222> 1..18
   <223> /mol_type="DNA" /note="oligonucleotide" /organism="Artificial"
<400> 11
   ggctccagct tccaggct 18
<210> 12
   <211> 15
   <212> DNA
   <213> Artificial
<220>
   <221> source
   <222> 1..15
   <223> /mol_type="DNA" /note="oligonucleotide" /organism="Artificial"
<400> 12
   tccagcttcc aggct 15